**Europäisches Patentamt**

**European Patent Office**

**Office européen des brevets**

(11) Publication number: **0 180 968**
**A2**

# (12) EUROPEAN PATENT APPLICATION

(21) Application number: **85114086.3**

(22) Date of filing: **05.11.85**

(51) Int. Cl.⁴: **A 61 K 7/48**

(30) Priority: **06.11.84 US 668667**

(43) Date of publication of application:
**14.05.86 Bulletin 86/20**

(84) Designated Contracting States:
**AT BE CH DE FR GB IT LI LU NL SE**

(71) Applicant: **Exovir, Inc.**
**10 Cutter Mill Road**
**Great Neck New York 11021(US)**

(72) Inventor: **Miller, Daniel G.**
**6 Fox Meadow Road**
**Scarsdale New York 10583(US)**

(74) Representative: **Vossius Vossius Tauchner Heunemann Rauh**
**Siebertstrasse 4 P.O. Box 86 07 67**
**D-8000 München 86(DE)**

(54) Antiwrinkle cosmetic preparation.

(57) Human serum albumin is useful as a skin antiwrinkle agent. More particularly, a composition comrpising human serum albumin, an aqueous carrier therefor including, preferably, a preservative, is useful as a skin antiwrinkle agent, particularly for temporarily smoothing facial wrinkles, such as the fine wrinkles around the eyes, forehead, mouth, neck and cheeks.

EP 0 180 968 A2

Our Ref: U 100 EP
Exovir, Inc.
Case: 22625-EPO

**0180968**

VOSSIUS · VOSSIUS · TAUCHNER
HEUNEMANN · RAUH
PATENTANWÄLTE
SIEBERTSTR. 4, 8000 MÜNCHEN 80
TEL. (089) 47 40 75

November 5, 1985

## ANTIWRINKLE COSMETIC PREPARATION

Certain fractions of albumen, particularly raw albumen from fresh whites of eggs, have been incorporated as an active ingredient in facial cosmetic materials for skin smoothing and to give an appearance of a more youthful, fresh and firm skin, see U.S. Patent 2,043,657. A steroid-hormone-free albumin-containing substance has been disclosed to be useful for application to the skin surface in a suitable vehicle to reduce skin folds and wrinkles and to cause the skin surface to have a more youthful appearance, see U.S. Patent 3,041,245. Albumin-containing compositions, particularly bovine serum albumin compositions and albumin derived from swine ovaries and placentas, have also been disclosed as useful for the temporary smoothing of facial wrinkles, see the article of A. M. Kligman et al entitled "Albumin as an Antiwrinkling Cosmetic", J. Soc. Cosmetic Chemists, 16, 557-562 (1965). The disclosures of the above-identified publications are herein incorporated and made part of this disclosure.

For the most part, however, previously known formulations proposed as antiwrinkle cosmetic formulations have not been completely satisfactory. For example, beef or bovine albumin, when applied to the face or skin as an antiwrinkle agent, sometimes produces an allergic reaction. Other albumin-containing formulations are not consistently effective because of variations in the methods of producing such albumin-containing formulations with resulting variations in effectiveness due to changes in physical and chemical compositions.

It is an object of this invention to provide an antiwrinkle cosmetic formulation useful and effective for its intended purposes.

It is another object of this invention to provide an antiwrinkle albumin-containing formulation which is substantially non-allergenic, when applied to the skin, particularly facial skin, to effect smoothing of the skin and removal of fine wrinkles.

How these and other objects of this invention are achieved will become apparent in the light of the accompanying disclosure.

Human serum albumin (HSA) has been found to be useful as an antiwrinkle or anti-wrinkling agent for the smoothing of skin and the temporary removal of fine wrinkles and the like. Human serum albumin is a well-known material and has a molecular weight of about 64,500, a diffusion constant $(D \times 10^7)$ of about 6.1, a sedimentation constant (S) of about 4.6 and a pI (isoelectric) value of about 4.8.

Human serum albumin employed in the practices of this invention can be obtained from and derived by processing human serum or obtained by expressing a cloned gene for human serum albumin.

Cosmetic compositions or materials containing human serum albumin as an antiwrinkle agent contain an effective skin smoothing or antiwrinkle amount of human serum albumin, usually a minor amount in the range from about 5 to about 50% by weight. A particular useful composition contains about 30% by weight.

The human serum albumin containing cosmetic preparations in accordance with this invention usually contain the human serum albumin in an aqueous medium or other physiologically acceptable carrier which does not cause precipitation or denaturing of the albumin and which is suitable for application to the skin area to be smoothed, particularly facial skin. A useful aqueous carrier for human serum albumin in the preparation of such compositions is rosewater. Rosewater is an aqueous solution with rose scent produced by the steam distillation of fresh flowers of rose plants. Desirably, there is also included in the carrier, such as an aqueous carrier, for the human serum albumin, e.g. rosewater, a small amount of glycerine or other polyhydric alcohol or glycol, e.g. about 0.5-2% weight glycerine, such as 1% by weight, and also desirably a preservative for the human serum albumin, e.g. Paraben.

The antiwrinkle cosmetic preparation prepared in accordance with this invention should desirably be kept prior to use at substantially ambient temperature, preferably in the range about 0-25°C., preferably in the range about 5-20°C.

When the antiwrinkle preparation is applied to the skin to effect skin smoothing and fine wrinkle removal, a small amount of the liquid preparation is applied to the skin to be treated and smoothed thereon to provide thereon a uniform liquid film which is then permitted to dry. During drying, the resulting contraction tension tends to pull the surrounding skin which usually yields little. Instead, the drying and dried lotion lifts the skin up and the fine wrinkles or furrows therebeneath are, in effect, lifted to the normal skin surface, thereby smoothing out and, in effect, removing the fine wrinkles. The overall effect as a result is simply lifting the wrinkles to the level of the surrounding skin, a smoothing action. The important advantages of human serum albumin as the antiwrinkle agent in the formulation is that it blends almost imperceptibly with the skin and does not form a visible plastic-like sheet thereon.

Still another interesting advantage of the use of human serum albumin as an antiwrinkle agent is that after the human serum albumin antiwrinkle preparation or liquid has been applied to the skin and dried, skin creams and coloring materials and other cosmetic preparations can be applied directly over the dried antiwrinkle lotion.

EXAMPLE

An antiwrinkle lotion is prepared by admixing with rosewater containing glycerine and a preservative, such as Paraben, a suitable amount of human serum albumin. The resulting admixture at a temperature of about 10-20°C. is stirred to provide a homogeneous solution. The amounts of the materials employed in the preparation of this antiwrinkle lotion are sufficient such that HSA comprises about 30% by weight thereof, glycerine comprises about 1% by weight thereof, and the remainder rosewater, about 70% by weight, together with a very minor amount of a perservative, such as Paraben.

0180968

In use as an antiwrinkle agent, before the HSA preparation is applied to the skin, the skin is cleansed and dried, such as by cleansing with soap and water or by use of a cleansing cream. A small amount of the HSA or solution is lightly brushed upon the skin to be treated for the removal of fine wrinkles and the like so as to form a smooth, homogeneous, transparent film thereon. The applied HSA preparation is then permitted to dry on the skin with the result that the fine wrinkles and the like upon which the lotion is applied disappear and the skin assumes a youthful smooth appearance. If desired, after the HSA preparation has dried on the skin, various cosmetic preparations, e.g. creams, coloring agents, and other cosmetic materials, including powders, can be applied for cosmetic purposes without adversely affecting the HSA coated, now substantially wrinkle-free, skin.

As will be apparent to those skilled in the art in the light of the foreoging, disclosure, many modifications, substitutions and alterations are possible in the practices of this invention without departing from the spirit or scope thereof.

0180968

<u>P a t e n t   C l a i m s</u>

1. A composition useful as an antiwrinkle cosmetic material comprising human serum albumin, an aqueous carrier therefor, and a preservative for said human serum albumin.

2. A composition in accordance with Claim 1 wherein said human serum albumin is present in a minor amount by weight.

3. A composition in accordance with Claim 1 wherein said human serum albumin is present in said composition in an amount in the range 5-50%.

4. A composition in accordance with Claim 1 wherein said human serum albumin is present in an amount of about 30% by weight.

5. A composition in accordance with Claim 1, wherein said aqueous carrier is rosewater.

6. A composition in accordance with Claim 1 wherein said aqueous carrier comprises rosewater and glycerine, the glycerine being present in said composition in an amount in the range about 0.5-2% by weight.

7. A composition in accordance with Claim 1 wherein said human serum albumin is derived from human serum.

8. A composition in accordance with Claim 1 wherein said human serum albumin is derived by expressing a cloned gene for human serum albumin.

0180968

9. A method of smoothing out the skin to temporarily remove fine wrinkles which comprises applying to the skin an effective amount of a composition in accordance with Claim 1.

10. The use of human serum albumin for the preparation of a composition useful as an antiwrinkle cosmetic material.